# FASCICULE DE BREVET EUROPEEN

(11) **EP 1 894 000 B1**
(45) Date de publication et mention de la délivrance du brevet: **20.06.2012**
(21) Numéro de dépôt: 06778627.7
(22) Date de dépôt: 20.06.2006
(51) Int. Cl.: C12Q 1/04, C12Q 1/22, G01N 33/96, G01N 1/00, G01N 1/28, A61M 1/02

(54) **PROCÉDÉ POUR LA DÉTERMINATION D'UNE CONTAMINATION BACTÉRIENNE DANS UN FLUIDE CONTENANT DES PLAQUETTES SANGUINES**
VERFAHREN ZUR BESTIMMUNG EINER BAKTERIELLEN KONTAMINATION IN EINER BLUTPLÄTTCHEN ENTHALTENDEN FLÜSSIGKEIT
METHOD FOR DETERMINING A BACTERIAL CONTAMINATION IN A BLOOD PLATELET-CONTAINING FLUID

(30) Priorité: 21.06.2005 FR 0506296
(43) Date de publication de la demande: 05.03.2008
(73) Titulaire: MACO PHARMA, 59420 Mouvaux (FR)
(72) Inventeur: H.WALKER, Wolfram, 634322 Rödermark (DE); GOUDALIEZ, Francis, F-59155 Faches-Thumesnil (FR); VERPOORT, Thierry, F-59250 Halluin (FR)
(74) Mandataire: Herrou, Nathalie
(86) Numéro de dépôt international: PCT/FR2006/001397
(87) Numéro de publication internationale: WO 2006/136698

(56) Documents cités:
- WO-A-97/43452
- WO-A2-96/37177
- WO-A2-2004/105837
- HISHON M L ET AL: "An evaluation of changes in composition and contamination of salvaged blood from the cardiopulmonary bypass circuit of pediatric patients." HEART & LUNG : THE JOURNAL OF CRITICAL CARE, vol. 24, no. 4, juillet 1995 (1995-07), pages 307-311, XP007900301 ISSN: 0147-9563

## Description

L'invention concerne un procédé pour la détermination d'une contamination pathogène, notamment bactérienne, dans un fluide contenant des plaquettes sanguines.

L'invention s'applique notamment à la détermination des bactéries susceptibles d'être présentes dans un concentré plaquettaire issu d'un don de sang total ou d'une procédure d'aphérèse.

Comme tous les produits sanguins labiles tels que les concentrés de globules rouges ou le plasma, les concentrés plaquettaires peuvent être contaminés par des pathogènes, tels que des virus, des parasites et notamment des bactéries. La transfusion à un patient de concentrés plaquettaires contaminés par des bactéries peut engendrer une septicémie chez le patient transfusé.

Pour éviter ce problème, des échantillons des concentrés plaquettaires à transfuser sont prélevés et analysés afin de détecter l'éventuelle présence de bactéries. La détection est réalisée soit au moment de la préparation des concentrés plaquettaires (détection précoce), soit après une période de conservation afin de s'assurer de l'absence de bactéries dans le produit à transfuser (détection tardive).

Cependant, si la contamination est très faible, elle peut ne pas être détectable au moment de la préparation des concentrés plaquettaires et un risque existe de transfuser à un patient un concentré plaquettaire contaminé.

D'un autre point de vue, si la détection est réalisée après une période de conservation, des plaquettes contaminées peuvent avoir été conservées inutilement, puisqu'elles ne seront pas transfusées.

Par exemple, le document WO 96/37177 décrit un système constitué d'une chambre primaire destinée au stockage d'un fluide biologique tel qu'un concentré plaquettaire, en communication fluidique avec deux chambres secondaires contenant chacune un réactif. Les deux chambres secondaires sont prévues pour détecter des contaminants différents (leucocytes et bactéries) ou pour effectuer deux fois le même test dans le but de confirmer le premier test. La détection des contaminants est réalisée juste avant l'utilisation du fluide, c'est-à-dire de façon tardive, et sans stockage des échantillons.

Le document WO 97/43452 décrit une méthode pour déterminer une contamination virale dans des dons de plasma stockés dans un récipient auquel est connectée une tubulure. Pour réaliser l'échantillonnage, une partie du plasma est envoyée dans la tubulure puis celle-ci est soudée à plusieurs endroits pour former une pluralité de pochettes contenant des échantillons de plasma. La détection de la contamination virale est également effectuée de façon tardive uniquement au moment d'assembler ensemble plusieurs dons de plasma.

Dans le document Hishon et al., 1995, Heart&Lung, 24(4), 307-311, la contamination bactérienne d'un sang issu d'une circulation extracorporelle est testée lors de sa conservation à 18°C, à 6h et à 18h. Les échantillons sont prélevés au moment du test de contamination et ne sont donc pas stockés avant le test.

L'invention propose donc un procédé de détermination d'une contamination pathogène, notamment bactérienne, dans un fluide biologique contenant des plaquettes sanguines, permettant de remédier à ces inconvénients. Le procédé vise à réaliser au moins deux échantillonnages des concentrés plaquettaires. Le premier échantillon vise à réaliser une détection bactérienne précoce, c'est-à-dire au moment de la préparation des concentrés plaquettaires. Le second échantillon permet de réaliser une détection bactérienne tardive, c'est-à-dire après une période de conservation des concentrés plaquettaires et avant la transfusion au patient.

Cette détection tardive laisse le temps aux bactéries éventuellement présentes de se multiplier et de pouvoir, le cas échéant, être détectées plus facilement.

A cet effet, l'invention porte sur un procédé de détermination d'une contamination bactérienne, dans un fluide biologique contenant des plaquettes sanguines, ledit procédé comprenant les étapes prévoyant de :
- préparer ou introduire le fluide biologique dans une poche de conservation ;
- prélever deux échantillons du fluide biologique présent dans la poche de conservation ;
- séparer le contenu de la poche et les deux échantillons ;
- déterminer la contamination bactérienne du premier échantillon à un instant initial ;
ledit procédé prévoyant, lorsque la contamination bactérienne du premier échantillon est négative, de :
- stocker la poche de conservation contenant le fluide biologique dans des premières conditions de stockage ;
- stocker le deuxième échantillon dans des deuxièmes conditions de stockage ;
- avant l'utilisation du fluide biologique, déterminer la contamination bactérienne du deuxième échantillon ;
- utiliser le fluide biologique en cas de contamination bactérienne négative du deuxième échantillon.

L'invention sera comprise grâce à la description qui suit en référence aux dessins annexés, illustrant divers modes de réalisation.
Les figures 1 à 3 et 8 représentent de façon schématique des systèmes à poches destinés à mettre en oeuvre le procédé de détermination d'une contamination bactérienne selon l'invention, ledit système comprenant une seule poche d'échantillonnage.
Les figures 4 à 7 représentent de façon schématique des systèmes à poches destinés à mettre en oeuvre le procédé de détermination d'une contamination bactérienne selon l'invention, ledit système comprenant deux poches d'échantillonnage.
La figure 9 représente de façon schématique un système à poches destiné à inactiver des pathogènes d'un concentré plaquettaire et à mettre en oeuvre le procédé de détermination d'une contamination bactérienne selon l'invention.

L'invention propose un procédé de détermination d'une contamination pathogène, notamment bactérienne, dans un fluide biologique contenant des plaquettes sanguines.

Le fluide biologique est notamment un concentré plaquettaire issu d'un ou plusieurs dons de sang total ou un concentré plaquettaire obtenu par aphérèse (CPA).

Selon une réalisation, le concentré plaquettaire est additionné d'une solution de conservation des plaquettes. Une telle solution est par exemple la solution SSP (Maco Pharma, Tourcoing, France).

Selon une autre réalisation, le concentré plaquettaire est déleucocyté, en particulier par filtration, préalablement à sa conservation.

En variante, le concentré plaquettaire a subi une procédure d'inactivation des pathogènes telle que décrite dans les documents WO-02/02152 ou WO-2005/004862, préalablement à sa conservation.

Ce concentré plaquettaire est destiné à être transfusé à un patient et est généralement conservé à température ambiante, environ 22°C, et jusqu'à 5 jours avant son utilisation. Cette température de conservation favorisant la prolifération bactérienne, le risque est d'autant plus important de transfuser à un patient un fluide contaminé et la détermination d'une contamination bactérienne s'avère essentielle.

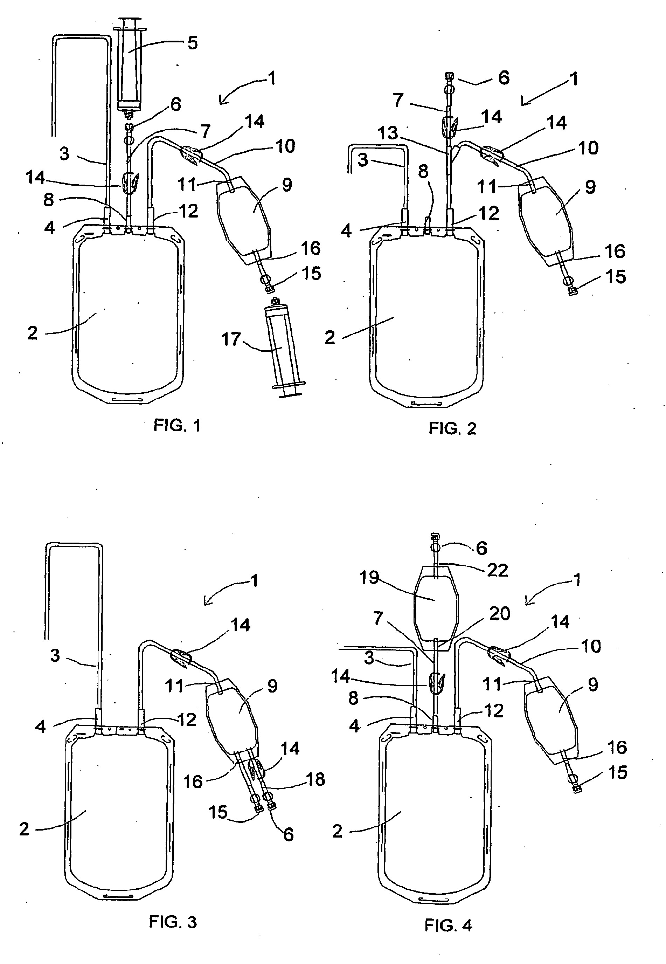

En relation avec la figure 1, on décrit maintenant un premier mode de réalisation d'un procédé de détermination d'une contamination pathogène, notamment bactérienne, selon l'invention ainsi qu'un système à poches 1 pour mettre en oeuvre ce procédé.

Pour déterminer la contamination bactérienne, le procédé selon l'invention comprend d'abord l'étape de préparer ou introduire le fluide biologique dans une poche de conservation 2.

Dans le cas où le fluide biologique est un concentré plaquettaire, il est préparé par exemple, directement dans la poche de conservation 2, en particulier par centrifugation d'un plasma riche en plaquettes (PRP) contenu dans ladite poche de conservation puis retrait de la poche de conservation de la couche surnageante ainsi obtenue et constituée de plasma.

En variante, le concentré plaquettaire est introduit directement dans la poche de conservation 2. Par exemple, le concentré plaquettaire est préparé par centrifugation à partir d'une unité de sang total, à l'aide d'un sous-ensemble à poches, ledit sous-ensemble étant connecté à une tubulure 3 reliée à l'orifice d'entrée 4 de la poche de conservation 2.

La poche de conservation 2 est notamment réalisée dans un matériau choisi parmi le PVC plastifié avec du tri-octyl trimellilate (TOTM), l'éthylène-vinylacétate (EVA) ou une polyoléfine telle que le polypropylène. Ces matériaux ont une perméabilité à l'oxygène accrue par rapport à un PVC plastifié DEHP utilisé couramment dans les systèmes à poches destiné au traitement du sang.

Une fois le fluide dans la poche de conservation 2, on prélève deux échantillons du fluide biologique présent dans la poche de conservation 2.

Par exemple, le premier échantillon est prélevé dans un récipient 5 adapté pour la réalisation de la détermination bactérienne.

Selon la figure 1, le premier échantillon est prélevé de la poche de conservation 2 dans un récipient 5 adapté à la détermination bactérienne, de type seringue munie d'un embout Luer, par connexion de ladite seringue à un dispositif de transfert de type connecteur Luer 6.

Ces connexions de type Luer-Luer présentent l'avantage d'être fixes et réversibles et évitent l'emploi d'aiguille et donc le risque de piqûre.

Le connecteur Luer 6 est relié par l'intermédiaire d'une première tubulure 7 à un orifice de sortie 8 de la poche de conservation 2.

Le deuxième échantillon est quant à lui prélevé dans une poche d'échantillonnage 9 connectée à la poche de conservation 2.

Selon la figure 1, la poche d'échantillonnage 9 est reliée, par l'intermédiaire d'une deuxième tubulure 10 et au niveau d'un orifice d'entrée 11 de la poche d'échantillonnage 9, à un second orifice de sortie 12 de la poche de conservation 2.

En variante représentée sur la figure 2, la poche d'échantillonnage 9 est reliée, par l'intermédiaire d'une deuxième tubulure 10 et au niveau d'un orifice d'entrée 11 de la poche d'échantillonnage 9, à un connecteur 13 prévu sur la première tubulure 7.

Le connecteur 13 est par exemple un connecteur trois voies en forme de T ou Y.

Le prélèvement du deuxième échantillon est réalisé au même moment que le prélèvement du premier échantillon ou ultérieurement avant l'utilisation du fluide.

Dès les échantillons prélevés, on sépare le contenu de la poche 2 et les deux échantillons, de sorte à couper la communication fluidique entre la poche de conservation 2 et les premier et deuxième échantillons, respectivement.

La séparation est effectuée par fermeture d'un dispositif sélectif de fermeture de type clamp 14 disposé, par exemple, sur la première et/ou la deuxième tubulure 7, 10. En variante, la séparation est effectuée par soudure et coupure de la première et/ou de la deuxième tubulure 7, 10.

Lorsque le prélèvement de l'échantillon nécessite de mettre le système à poches en contact avec l'extérieur, comme dans le cas d'un transfert de fluide dans un tube ou une seringue non pré-connecté, cette séparation des échantillons vise à prévenir une contamination ultérieure de la poche de conservation 2 et/ou de la poche d'échantillonnage 9 contenant le deuxième échantillon.

On détermine la contamination bactérienne du premier échantillon à un instant initial, notamment au moment où le fluide est préparé ou introduit dans la poche de conservation 2.

Dans le mode de réalisation de la figure 1, cette détermination de la contamination bactérienne est réalisée au moment du prélèvement du premier échantillon dans le récipient 5.

Cette première détermination, dite précoce, est effectuée par exemple par mise en culture de l'échantillon présent dans le récipient adapté à la détermination de la contamination afin de faire proliférer les bactéries (système BacT/alert®) ou par addition à l'échantillon d'un colorant spécifique afin de colorer les bactéries (système FACS).

A cet effet, le récipient 5 est avantageusement pré-rempli avec les réactifs tels que le milieu de culture ou le colorant, nécessaires à la détermination bactérienne.

Si à l'instant initial, il est déterminé que la poche de conservation 2 contient un fluide biologique contaminé, alors la poche est jetée. Ainsi, la poche de conservation 2 n'est pas conservée inutilement.

Lorsque la contamination bactérienne du premier échantillon est négative, on stocke la poche de conservation 2 contenant le fluide biologique dans des premières conditions de stockage.

Dans le cas d'un concentré plaquettaire, le fluide est conservé à température ambiante, c'est-à-dire environ 22°C.

On stocke également le deuxième échantillon dans des deuxièmes conditions de stockage.

Selon une réalisation du procédé, les première et deuxième conditions de stockage sont identiques, c'est-à-dire dans le cas d'un concentré plaquettaire, le fluide et le deuxième échantillon sont conservés à température ambiante, c'est-à-dire environ 22°C.

Ainsi, la contamination bactérienne de l'échantillon après conservation correspond à celle de la poche de conservation du fluide 2.

Selon la figure 1, le stockage du deuxième échantillon est réalisé dans la poche d'échantillonnage 9. Cette poche est réalisée notamment dans les mêmes matériaux que la poche de conservation 2.

Selon une réalisation particulière, la poche d'échantillonnage 9 est séparée de la poche de conservation 2 préalablement au stockage. Cette séparation réalisée par exemple par soudure et coupure de la deuxième tubulure 10, permet de stocker le deuxième échantillon dans des deuxièmes conditions de stockage indépendantes des premières conditions de stockage de la poche de conservation 2.

Ainsi selon une autre réalisation du procédé, les première et deuxième conditions de stockage peuvent être différentes concernant la température de stockage.

Par exemple, la température de stockage du fluide biologique est inférieure à la température de stockage du deuxième échantillon.

En effet, afin de stimuler la croissance bactérienne, le deuxième échantillon peut être conservé à une température d'environ 37°C, le fluide étant conservé à environ 22°C.

Les dernières étapes du procédé selon l'invention prévoient, avant l'utilisation du fluide biologique, de déterminer la contamination bactérienne du deuxième échantillon, et d'utiliser le fluide biologique en cas de contamination bactérienne négative du deuxième échantillon.

En particulier, la détermination de la contamination bactérienne du deuxième échantillon est réalisée par transfert dudit échantillon de la poche d'échantillonnage 9 dans un récipient 17 adapté pour la réalisation de ladite détermination.

Selon la figure 1, le transfert est réalisé par l'intermédiaire d'un dispositif de transfert de type connecteur Luer 15 relié au niveau d'un orifice de sortie 16 de la poche d'échantillonnage 9 contenant le deuxième échantillon. Le récipient 17 est dans ce cas une seringue munie d'un embout Luer que l'on vient connecter au Luer 15.

Cette deuxième détermination, dite tardive, est réalisée après la détermination du premier échantillon, notamment entre 8h et 5 jours après la préparation ou l'introduction du fluide biologique dans la poche de conservation 2.

Cette détermination tardive, effectuée par les méthodes de détermination citées ci-dessus, permet de détecter une contamination trop faible pour être détectée au moment de la détermination précoce.

Par exemple, si la contamination a eu lieu au moment de la préparation ou l'introduction du fluide dans la poche de conservation 2, elle peut être au-dessous du seuil de détection des méthodes de détermination d'une contamination bactérienne.

On décrit maintenant des variantes du procédé de détermination de la contamination pathogène, notamment bactérienne.

Selon le mode de réalisation représenté sur la figure 3, au lieu d'être réalisé dans la poche de conservation 2, le prélèvement du premier échantillon est réalisé dans la poche d'échantillonnage 9 utilisée pour le stockage du deuxième échantillon.

Dans ce cas, la détermination précoce du premier échantillon est effectuée à l'aide d'un dispositif de transfert, distinct du dispositif de transfert du deuxième échantillon, disposé par exemple au niveau d'un autre orifice de sortie de la poche d'échantillonnage 9.

Ainsi, le prélèvement du deuxième échantillon est réalisé à l'aide d'un dispositif de transfert non encore utilisé, et qui n'a donc pas été soumis à un risque de contamination lors de la détermination d'un premier échantillon, notamment lors de la connexion et/ou déconnexion du récipient adapté à la détermination de la contamination.

Par exemple, le dispositif de transfert, notamment un Luer 6 est relié par l'intermédiaire d'une tubulure 18 munie d'un clamp 14 à l'autre orifice de sortie de la poche d'échantillonnage 9. Ce clamp 14 est fermé après la détermination de l'échantillon pour prévenir le risque de contamination de la poche d'échantillonnage 9 après prélèvement du premier échantillon.

Dans ce mode de réalisation, le prélèvement et la détermination de la contamination du deuxième échantillon sont réalisés comme décrit en relation avec la figure 1.

En relation avec la figure 4 et selon un autre mode de réalisation, le premier échantillon est prélevé dans une poche d'échantillonnage 19 préalablement à la détermination de sa contamination bactérienne, ladite poche étant connectée à la poche de conservation 2.

Cette poche d'échantillonnage 19 pour le premier échantillon est distincte de la poche d'échantillonnage 9 dans laquelle est stockée le deuxième échantillon.

Comme illustrée sur la figure 4, la poche d'échantillonnage 19 pour le premier échantillon est reliée par l'intermédiaire d'une première tubulure 7 et au niveau d'un orifice d'entrée 20, à un premier orifice de sortie 8 de la poche de conservation 2.

La poche d'échantillonnage 9 pour le deuxième échantillon est comme décrite en relation avec la figure 1 ou 2.

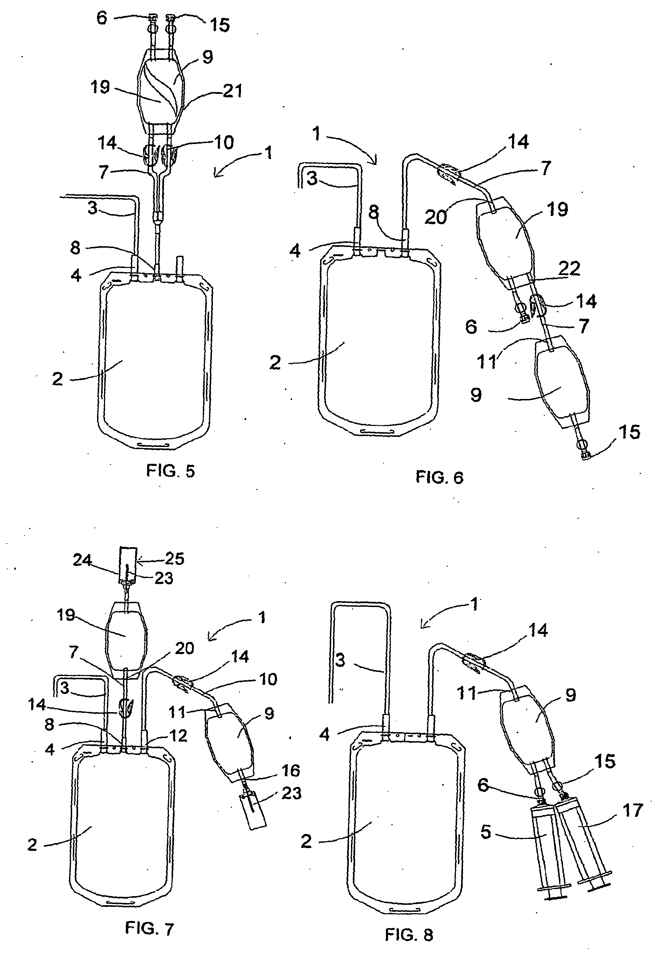

Selon une autre variante de ce mode de réalisation illustrée sur la figure 5, les deux poches d'échantillonnage 9, 19 pour le premier et le deuxième échantillons sont associées de sorte à former une poche siamoise 21 telle que décrite dans document EP-A1-0 084 512.

La détermination d'une contamination bactérienne des premier et deuxième échantillons est effectuée par transfert desdits échantillons dans un premier et deuxième récipients 5, 17 adaptés pour la réalisation de cette détermination.

En relation avec la figure 6 et selon un mode de réalisation particulier, le premier échantillon est prélevé dans une poche d'échantillonnage 19 préalablement à la détermination de sa contamination bactérienne, ladite poche étant connectée à la poche de conservation 2, et le deuxième échantillon est prélevé dans une poche d'échantillonnage 9 connectée à la poche d'échantillonnage 19 du premier échantillon.

Notamment, la poche d'échantillonnage 19 du premier échantillon est reliée, par l'intermédiaire d'une première tubulure 7 et au niveau d'un orifice d'entrée 20, à un orifice de sortie 8 de la poche de conservation 2 du fluide. Et la poche d'échantillonnage 9 du deuxième échantillon est reliée, par l'intermédiaire d'une deuxième tubulure 10 et au niveau d'un orifice d'entrée 11, à un orifice de sortie 22 de la poche d'échantillonnage 19 du premier échantillon.

En variante de cette réalisation (non représentée), les poches d'échantillonnage du premier et deuxième échantillons sont inversées, c'est-à-dire le premier échantillon est prélevé dans une poche d'échantillonnage connectée à la poche d'échantillonnage du second échantillon, elle même connectée à la poche de conservation.

La détermination d'une contamination bactérienne des premier et deuxième échantillons est effectuée par transfert desdits échantillons dans un premier et deuxième récipients 5, 17 adaptés pour la réalisation de cette détermination.

Les transferts sont effectués à l'aide des dispositifs de transferts de types connecteurs Luer, par exemple reliés aux poches d'échantillonnage 9, 19.

Dans ces différents mode de réalisation d'un système à poches utilisé dans le procédé selon l'invention (figures 1 à 6), chaque tubulure peut être munie d'un moyen sélectif de fermeture de la communication fluidique au travers de ladite tubulure. Ce moyen sélectif est notamment un clamp à fermeture réversible 14 ou irréversible, et permet d'assurer la non contamination du système à poches.

En outre, le transfert des échantillons dans des récipients 5, 17 adaptés à la détermination d'une contamination bactérienne est réalisé à l'aide d'un dispositif de transfert, typiquement des connecteurs de type Luer 6, 15 auxquels peuvent être connectés des seringues à embout Luer formant récipients 5, 17.

En variante et comme illustré sur la figure 7, le dispositif de transfert est une aiguille creuse 23 ou un perforateur (non représenté), éventuellement entouré(e) d'un tube creux 24, de sorte à former un porte-tube 25. La détermination bactérienne est alors réalisée dans des récipients de type tubes sous vide, munis de bouchons perforables par l'aiguille creuse ou le perforateur 23.

Selon un aspect particulier, les différents éléments du système à poches sont pré-connectés de fabrication, de sorte à former un système clos.

Selon un autre aspect, comme illustré par exemple sur la figure 8, les récipients 5, 17 adaptés à la détermination de la contamination bactériennes, par exemple les seringues, sont aussi pré-connectées aux dispositifs de transferts, par exemple, les Luer 6, 15. Ces seringues contiennent avantageusement les réactifs nécessaires à une détermination bactérienne.

Dans ce cas, le prélèvement et la détermination des échantillons sont réalisés de façon stérile, réduisant les risques de contamination de la poche de conservation 2 ou des poches d'échantillonnage 9, 19, puisque le système à poches reste clos ou fermé lors de ces deux procédures de prélèvement et de détermination.

Selon un aspect particulier, chacun de ces différents systèmes à poches est connecté ou destiné à être connecté à un sous-ensemble à poches destiné au traitement du sang, notamment la séparation d'une unité de sang total en les différents constituants du sang, le pooling de buffy coat ou de concentrés plaquettaires, ou encore l'inactivation des pathogènes du sang, par exemple comme décrit dans les documents WO-02/02152 ou WO-2005/004862.

Par exemple, la tubulure 3 reliée à l'orifice d'entrée 4 de la poche de conservation 2 est munie d'un perforateur pour permettre la connexion à autre une poche (non représentée).

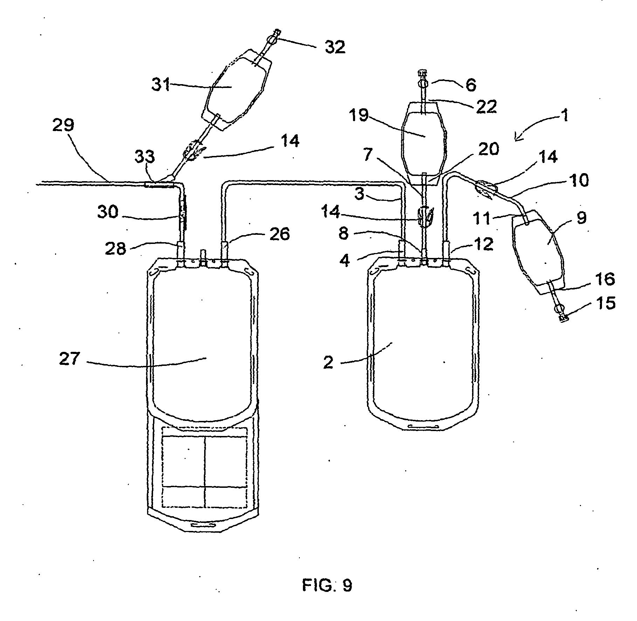

Selon un autre exemple représenté sur la figure 9, la poche de conservation 2 est reliée par l'intermédiaire d'une tubulure 3 et au niveau d'un orifice d'entrée 4 à l'orifice de sortie 26 d'une poche d'illumination 27. Un orifice d'entrée 28 de ladite poche d'illumination 27 est reliée à une tubulure 29 comprenant une substance inactivante 30, comme par exemple le 5-ALA ou la thionine.

Le système à poches comprend en outre un dispositif d'échantillonnage comprenant une poche d'échantillonnage 31 muni d'un dispositif de transfert 32 vers un récipient de détermination d'une contamination, ladite poche d'échantillonnage 31 étant reliée au niveau d'un connecteur 33 pourvu sur ladite tubulure 29 reliée à la poche d'illumination 30, le connecteur 33 étant placé en amont de la substance inactivante dans le sens normal d'écoulement des fluides dans le système.

Ce dispositif d'échantillonnage 32 permet de réaliser une détection de la contamination bactérienne avant le traitement d'inactivation.

Le procédé de détermination de la contamination bactérienne selon l'invention est dans ce cas réalisé sur le fluide biologique ayant subi une inactivation des pathogènes préalablement à son introduction dans la poche de conservation.

Après inactivation, le fluide inactivé est envoyé dans la poche de conservation 2 et la détermination d'une contamination bactérienne est réalisée selon le procédé de l'invention.

## Revendications

1. Procédé de détermination d'une contamination bactérienne dans un fluide biologique contenant des plaquettes sanguines, ledit procédé comprenant les étapes prévoyant de :
- préparer ou introduire le fluide biologique dans une poche de conservation (2) ;
- prélever deux échantillons du fluide biologique présent dans la poche de conservation (2) ;
- séparer le contenu de la poche et les deux échantillons ;
- déterminer la contamination bactérienne du premier échantillon à un instant initial ;
ledit procédé prévoyant, lorsque la contamination bactérienne du premier échantillon est négative, de :
- stocker la poche de conservation (2) contenant le fluide biologique dans des premières conditions de stockage ;
- stocker le deuxième échantillon dans des deuxièmes conditions de stockage ;
- avant l'utilisation du fluide biologique, déterminer la contamination bactérienne du deuxième échantillon ;
- utiliser le fluide biologique en cas de contamination bactérienne négative du deuxième échantillon.

2. Procédé de détermination selon la revendication 1, dans lequel les premières et deuxièmes conditions de stockage sont identiques.

3. Procédé de détermination selon la revendication 1, dans lequel les températures respectives des premières et deuxièmes conditions de stockage sont différentes.

4. Procédé de détermination selon la revendication 3, dans lequel la température de stockage du fluide biologique est inférieure à la température de stockage du deuxième échantillon.

5. Procédé de détermination selon l'une quelconque des revendications 1 à 4, dans lequel le deuxième échantillon est prélevé dans une poche d'échantillonnage (9) connectée à la poche de conservation (2).

6. Procédé de détermination selon la revendication 5, dans lequel la poche d'échantillonnage (9) est séparée de la poche de conservation préalablement au stockage.

7. Procédé de détermination selon la revendication 5 ou 6, dans lequel la détermination de la contamination bactérienne du deuxième échantillon est réalisée par transfert dudit échantillon dans un récipient (17) adapté pour la réalisation de ladite détermination.

8. Procédé de détermination selon la revendication 5, dans lequel le prélèvement du premier échantillon est réalisé dans la poche d'échantillonnage (9).

9. Procédé de détermination selon l'une quelconque des revendications 1 à 7, dans lequel le premier échantillon est prélevé dans un récipient (5) adapté pour la réalisation de ladite détermination.

10. Procédé de détermination selon l'une quelconque des revendications 1 à 7, dans lequel le premier échantillon est prélevé dans une poche d'échantillonnage (19) préalablement à la détermination de sa contamination bactérienne, ladite poche étant connectée à la poche de conservation (2).

11. Procédé de détermination selon la revendication 10 lorsqu'elle ne dépend pas de la revendication 5, dans lequel le deuxième échantillon est prélevé dans une poche d'échantillonnage (9) connectée à la poche d'échantillonnage du premier échantillon (19).

12. Procédé de détermination selon l'une quelconque des revendications 1 à 11, dans lequel le fluide biologique a subi une inactivation bactérienne préalablement à son introduction dans la poche de conservation.

## Claims

1. A method for determining a bacterial contamination in a biological fluid containing blood platelets, said method comprising the following steps of:
- preparing or introducing the biological fluid into a storage bag (2);
- drawing two samples of the biological fluid present in the storage bag (2);
- separating the content of the bag and the two samples;
- determining the bacterial contamination of the first sample at a first moment;
said method, if the bacterial contamination of the first sample is negative, including the following steps of:
- storing the storage bag (2) containing the biological fluid under first storage conditions;
- storing the second sample under second storage conditions;
- determining the bacterial contamination of the second sample before the biological fluid is used;
- using the biological fluid in the case of negative bacterial contamination of the second sample.

2. The determination method according to claim 1, wherein the first and second storage conditions are identical.

3. The determination method according to claim 1, wherein the respective temperatures of the first and second storage conditions are different.

4. The determination method according to claim 3, wherein the storage temperature of the biological fluid is lower than the storage temperature of the second sample.

5. The determination method according to any one of claims 1 to 4, wherein the second sample is drawn into a sampling bag (9) connected to the storage bag (2).

6. The determination method according to claim 5, wherein the sampling bag (9) is separated from the storage bag prior to storage.

7. The determination method according to either claim 5 or claim 6, wherein the bacterial contamination of the second sample is determined by transferring said sample to a container (17) adapted for the execution of said determination.

8. The determination method according to claim 5, wherein the first sample is drawn into the sampling bag (9).

9. The determination method according to any one of claims 1 to 7, wherein the first sample is drawn into a container (5) adapted for the execution of said determination.

10. The determination method according to any one of claims 1 to 7, wherein the first sample is drawn into a sampling bag (19) prior to the determination of its bacterial contamination, said bag being connected to the storage bag (2).

11. The determination method according to claim 10, when not dependent on claim 5, wherein the second sample is drawn into a sampling bag (9) connected to the sampling bag of the first sample (19).

12. The determination method according to any one of claims 1 to 11, wherein the biological fluid has undergone a bacterial inactivation prior to its introduction into the storage bag.

## Patentansprüche

1. Verfahren zum Bestimmen einer bakteriellen Kontamination in einem biologischen Fluid, das Thrombozyten enthält, wobei das Verfahren die Schritte umfasst, die Folgendes vorsehen:
- Herstellen oder Einführen des biologischen Fluids in einen Konservierungsbeutel (2);
- Entnehmen von zwei Proben des biologischen Fluids, das in dem Konservierungsbeutel (2) vorhanden ist;
- Trennen des Beutelinhalts und der beiden Proben;
- Bestimmen der bakteriellen Kontamination der ersten Probe zu einem ersten Zeitpunkt;
wobei das Verfahren, wenn die bakterielle Kontamination der ersten Probe negativ ist, Folgendes vorsieht:
- Lagern des Konservierungsbeutels (2), der das biologische Fluid enthält, unter ersten Lagerungsbedingungen;
- Lagern der zweiten Probe unter zweiten Lagerungsbedingungen;
- Bestimmen der bakteriellen Kontamination der zweiten Probe vor der Verwendung des biologischen Fluids;
- Verwenden des biologischen Fluids im Fall einer negativen bakteriellen Kontamination der zweiten Probe.

2. Bestimmungsverfahren nach Anspruch 1, wobei die erste und die zweite Lagerungsbedingung identisch sind.

3. Bestimmungsverfahren nach Anspruch 1, wobei die jeweiligen Temperaturen der ersten und der zweiten Lagerbedingungen verschieden sind.

4. Bestimmungsverfahren nach Anspruch 3, wobei die Lagerungstemperatur des biologischen Fluids unterhalb der Lagerungstemperatur der zweiten Probe liegt.

5. Bestimmungsverfahren nach einem der Ansprüche 1 bis 4, wobei die zweite Probe in einem Probenentnahmebeutel (9) entnommen wird, der mit dem Konservierungsbeutel (2) verbunden ist.

6. Bestimmungsverfahren nach Anspruch 5, wobei der Probenentnahmebeutel (9) vor der Lagerung von dem Konservierungsbeutel getrennt wird.

7. Bestimmungsverfahren nach Anspruch 5 oder 6, wobei das Bestimmen der bakteriellen Kontamination der zweiten Probe durch Überführen der Probe in einen Behälter (17) durchgeführt wird, der für das Durchführen der Bestimmung geeignet ist.

8. Bestimmungsverfahren nach Anspruch 5, wobei das Entnehmen der ersten Probe in dem Probenentnahmebeutel (9) durchgeführt wird.

9. Bestimmungsverfahren nach einem der Ansprüche 1 bis 7, wobei die erste Probe in einem Behälter (5) entnommen wird, der für das Durchführen der Bestimmung geeignet ist

10. Bestimmungsverfahren nach einem der Ansprüche 1 bis 7, wobei die erste Probe vor dem Bestimmen ihrer bakteriellen Kontamination in einem Probenentnahmebeutel (19) entnommen wird, wobei der Beutel mit dem Konservierungsbeutel (2) verbunden ist.

11. Bestimmungsverfahren nach Anspruch 10, sofern er nicht von Anspruch 5 abhängig ist, wobei die zweite Probe in einem Probenentnahmebeutel (9) entnommen wird, der mit dem Probenentnahmebeutel der ersten Probe (19) verbunden ist.

12. Bestimmungsverfahren nach einem der Ansprüche 1 bis 11, wobei das biologische Fluid vor seiner Einführung in den Konservierungsbeutel einer bakteriellen Inaktivierung unterzogen wurde.
